# EUROPÄISCHE PATENTANMELDUNG

(11) **EP 2 930 192 A1**
(43) Veröffentlichungstag der Anmeldung: **14.10.2015**
(21) Anmeldenummer: 14164438.5
(22) Anmeldetag: 11.04.2014
(51) Int. Cl.: C08F 4/52, C08F 210/10, C07C 39/367

(54) **Initiator-System zur Herstellung von synthetischen Kautschuken**

(71) Anmelder: LANXESS Deutschland GmbH, 50569 Köln (DE)
(72) Erfinder: Höger, Sigurd, 53913 Swisttal-Morenhoven (DE); Le Blanc, Stephan, 53121 Bonn (DE); Paul, Hanns-Ingolf, 51375 Leverkusen (DE); Wiesner, Udo, 53332 Bornheim (DE); Leiberich, Ricarda, 63263 Neu-Isenburg (DE); Kirchhoff, Jörg, 51061 Köln (DE)

(57) **Zusammenfassung**

Die vorliegende Erfindung betrifft Initiator-Systeme für kationische Polymerisationen zur Herstellung von synthetischen Kautschuken wie insbesondere Polyisobutylen und ButylKautschuk.

## Beschreibung

Die vorliegende Erfindung betrifft Initiator-Systeme für kationische Polymerisationen zur Herstellung von synthetischen Kautschuken wie insbesondere Polyisobutylen und ButylKautschuk.

Butylkautschuk ist ein Copolymer aus Isobuten und typischerweise geringen Mengen an Isopren und wird in industriellen Verfahren üblicherweise bei Temperaturen unterhalb von -70°C kationisch polymerisiert.

Die Kinetik der kationischen Polymerisation von Olefinen wird dabei hauptsächlich bestimmt durch die Geschwindigkeitskonstanten von Kettenwachstum, Kation-Transfer und Termination. Die Wahl der Temperatur, des Lösungsmittels und des Initiator-Systems beeinflusst daher die Eigenschaften des hergestellten Butylkautschuks entscheidend.

Die tiefen Temperaturen machen den Herstellungsprozess in der Steuerung allerdings schwierig und energetisch unvorteilhaft.

Aus dem Stand der Technik sind verschiedene Initiator- Systeme bekannt.

Beispielsweise beschreibt WO2010/139684A1 Verbindungen aus einem protonierten Aromaten und einem schwach koordinierenden Anion der allgemeinen Formel HAr⁺An⁻, in der HAr⁺ eine protonierte, einkernige oder kondensierte mehrkernige aromatische Struktur mit einer oder mehreren C₁- bis C₄-Alkylgruppen als Substituenten bezeichnet, welche durch ein geeignetes Substitutionsmuster der C₁- bis C₄-Alkylgruppen eine Stabilisierung der delokalisierten positiven Ladung ermöglicht und An⁻ ein schwach oder nicht koordinierendes Anion bezeichnet.

Des Weiteren ist aus WO2006/011868A1, WO2004/067577A2 und WO2004/058835A1 prinzipiell bekannt, dass 2,3,4,5,6-Pentafluorphenol (PFP) für kationische Polymerisationen als Coinitiator einsetzbar ist. Als Lösungsmittel werden in vorgenannten Dokumenten Fluorkohlenwasserstoffe gegebenenfalls in Mischung mit Methylchlorid eingesetzt.

Der Stand der Technik zeigt insgesamt, dass es nicht ohne weiteres möglich ist, Elastomere wie insbesondere Polyisobutylen und Butylkautschuk mit einem hohen Molekülgewicht, bei moderaten Temperaturen und üblichen Lösungsmitteln mit jedem beliebigen Initiator- System herzustellen.

Aufgabe war es nun ein alternatives Initiator- System sowie ein Verfahren zur Herstellung von Elastomeren bereitzustellen, dass die Nachteile des Standes Technik überwindet.

Die Lösung der Aufgabe und Gegenstand der vorliegenden Erfindung ist nun ein Initiator-System umfassend
I) zumindest eine Verbindung der Formel (I)

   AY¹Y²Y³ (I)

   in der
   - A: für ein Metall aus der Gruppe IIIA, vorzugsweise Aluminium, Bor, Gallium oder Indium, besonders bevorzugt Bor und Aluminium und besonders bevorzugt Aluminium steht und
   - Y¹, Y² und Y³: jeweils unabhängig voneinander Fluor, Chlor, Brom, lod oder (C₁-C₁₈)-Alkyl stehen können und
II) zumindest eine Verbindung der Formeln (IIa) oder (IIb) 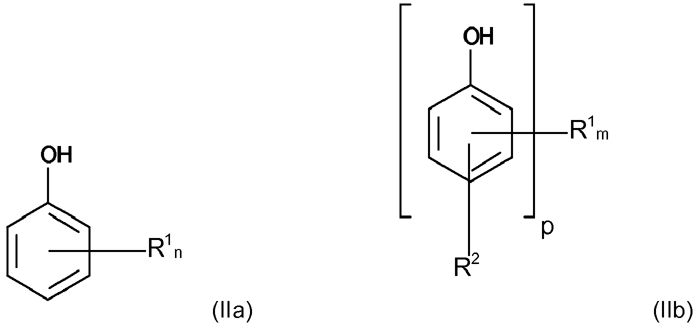 in denen
   - n: 1, 2, 3, 4 oder 5 und
   - m: 1, 2, 3 oder 4 und
   - p: 2, 3 oder 4 sein können
   - R¹: unabhängig von gegebenenfalls weiteren Resten R¹ ausgewählt ist aus der Gruppe Fluor, Chlor, Hydroxy, CN, NO₂, (C₁-C₁₈)-Alkyl, (C₁-C₁₈)-Alkoxy, (C₆-C₂₄)-Alkylaryl, (C₆-C₂₄)-Arylalkyl oder (C₆-C₁₀)-Aryl und
   - R²: für eine Bindung (in diesem Fall ist p =2) oder einen p-valenten Rest steht, der sich von Benzol, Naphthalin, Biphenyl, Terphenyl oder Tetraphenyl ableitet und der nicht, einfach oder mehrfach unabhängig weiter substituiert sein kann mit Resten aus der Gruppe Halogen, Hydroxy, CN, NO₂, (C₁-C₁₈)-Alkyl, (C₁-C₁₈)-Alkoxy, (C₆-C₂₄)-Alkylaryl oder (C₆-C₂₄)-Arylalkyl

Der Begriff des Initiator-System umfassend zumindest eine Verbindung der Formel (I) und zumindest eine Verbindung der Formel (II) umfasst auch Reaktionsprodukte, die durch Reaktion der vorgenannten Verbindungen der Formeln (I) und (II) typischerweise entstehen können. Dem Fachmann ist jedoch klar, dass aufgrund der Komplexität solcher Reaktionen und der Reaktionsprodukte die Angabe der Edukte für das Initiatorsystem ausreichend und zielführender ist.

Es sei an dieser Stelle angemerkt, dass der Rahmen der Erfindung alle beliebigen und möglichen Kombinationen der oben stehenden und im Folgenden aufgeführten, allgemeinen oder in Vorzugsbereichen genannten Komponenten, Wertebereiche bzw. Verfahrensparameter umfasst.

In Formel (I) stehen Y¹, Y² und Y³ bevorzugt jeweils unabhängig voneinander für Chlor oder (C₁-C₁₈)-Alkyl.

Besonders bevorzugt stehen Y¹, Y² und Y³ jeweils unabhängig voneinander Chlor oder C₁-C₄-Alkyl, insbesondere für Chlor, Methyl oder Ethyl und ganz besonders für Chlor oder Ethyl.

Besonders bevorzugte Verbindungen der Formel (I) sind Ethylaluminiumdichlorid (EADC) und Diethylaluminiumchlorid (DEAC) oder Mischungen davon. Ganz besonders bevorzugt ist Diethylaluminiumchlorid.

In den Formeln (IIa) und (IIb) steht R¹ bevorzugt für Fluor, Chlor, Hydroxy, (C₁-C₄)-Alkyl oder (C₁-C₄)-Alkoxy, besonders bevorzugt Fluor oder (C₁-C₄)-Alkyl, ganz besonders bevorzugt für Fluor.

Bevorzugt nimmt n die Zahl 2, 3, 4 oder 5, besonders bevorzugt 4 oder 5 an.

Eine bersonders bevorzugte verbindung der Formel (IIa) ist Pentafluorphenol.

Bevorzugt nimmt m die Zahl 2, 3 oder 4, besonders bevorzugt 3 oder 4 an.

In Formel (IIb) steht R² bevorzugt für eine Bindung oder für einen bivalenten 1,2- oder 1,4-Phenyl-Rest, wobei in einer bevorzugten Ausführungsform die Bindung zum oin Formel (IIb) dargestellten Aryl-Rest in ortho- oder para-Stellung, bevorzugt in para-Stellung zur dargestellten Hydroxygruppe erfolgt.

Eine besonders bevorzugte Verbindung ist 2,2"-Bis(2,3,5,6-tetrafluor-4-hydroxy)-para-terphenyl.

(C₁-C₁₈)-Alkyl bzw. (C₁-C₁₈)-Alkoxy, steht für einen geradkettigen, ab 3 Kohlenstoffatomen auch verzweigten oder cyclischen oder ab 4 Kohlenstoffatomen auch verzweigten cyclischen Alkyl- bzw. Alkoxy-rest mit einem bis 18 Kohlenstoffatomen. Die Definition gilt sinngemäß analog auch für (C₁-C₄)-Alkyl bzw. (C₁-C₄)-Alkoxy

(C₁-C₄)-Alkyl umfasst demzufolge Methyl, Ethyl, iso-Propyl, n-Butyl, sec-Butyl oder tert-Butyl, (C₁-C₈)-Alkyl darüber hinaus beispielsweise n-Hexyl, Cyclohexyl, iso-Octyl, n-Docecyl und n-Octadecyl.

(C₁-C₄)-Alkoxy umfasst demzufolge Methoxy, Ethoxy, n-Propoxy, Isopropoxy, n-Butoxy sec.-Butoxy und tert-Butoxy, (C₁-C₁₈)-Alkoxy darüber hinaus beispielsweise n-Pentoxy und n-Hexoxy.

C₆-C₂₄)-Arylalkyl steht beispielsweise für Benzyl, (C₆-C₂₄)-Alkylaryl beispielsweise für o-, m- oder p-tolyl, (C₆-C₁₀)-Aryl beispielsweise für Phenyl oder Naphthyl

Verbindungen der Formel (IIa) sind bekannt oder kommerziell erhältlich, Verbindungen der Formel (IIb) können beispielsweise in an sich bekannter Weise durch
A) Umsetzung von Verbindungen der Formel (III) 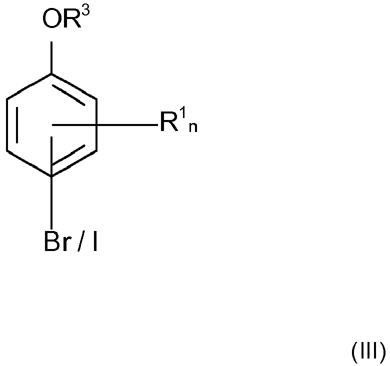 mit Verbindungen der Formel (IV) oder (V)

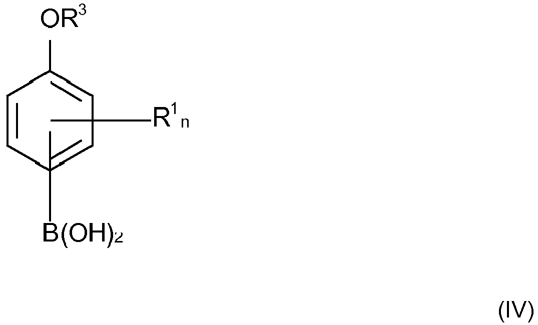

   R²[B(OH)₂]ₚ (V)

   in Gegenwart von Palladium enthaltenden Katalysatoren wie beispielsweise Tetrakis(triphenylphosphan)palladium(0) und anschließend
B) Spaltung der Ether-gruppen z.B. mit Bortribromid, Bortrichlorid oder Chlor- oder Bromwasserstoff.

Einige Verbindungen der Formeln (IIb) sind neu und daher von der Erfindung mitumfasst.

Die erfindungsgemäßen Initiator-Systeme eignen sich insbesondere zur Anwendung in einem Verfahren zur Herstellung von Homo- oder Copolymeren, die Wiederholungseinheiten aufweisen sich sich von Isoolefinen ableiten.

Die Erfindung umfasst daher ein Verfahren zur Herstellung von Polymeren, die Wiederholungseinheiten aufweisen sich sich von Isoolefinen ableiten, das zumindest die folgenden Schritte umfasst:
a) Bereitstellung eines Reaktionsmediums enthaltend ein organisches Verdünnungsmittel und zumindest Isoolefin als Monomer, vorzugsweise zumindest ein Isoolefin und zumindest ein Multiolefin als Monomere;
b) Polymerisation der Monomere im Reaktionsmedium in Gegenwart eines erfindungsgemäßen Initiator-Systems zur Erzeugung eines Produktmediums umfassend das Polymer, das organische Verdünnungsmittel und gegebenenfalls unreagierte Monomere und optional
c) Abtrennung des Polymers aus dem Produktmedium.

### Monomere

In Schritt a) erfolgt die Bereitstellung eines Reaktionsmediums enthaltend ein organisches Verdünnungsmittel und zumindest Isoolefin als Monomer, vorzugsweise zumindest ein Isoolefin und zumindest ein Multiolefin als Monomere.

Im Rahmen der Erfindung wird unter einem Isoolefin eine Verbindung verstanden, bei dem ein Kohlenstoffatom durch zwei Alkylreste substituiert ist und ein Kohlenstoffatom durch zwei Wasserstoffatome oder ein Wasserstoffatom und eine Alkylgruppe.

Beispiele von Isoolefinen umfassen Isobuten, 2-Methyl-1-buten, 3-Methyl-1-buten und 2-Methyl-2-buten. Besonders bevorzugt ist Isobuten.

Im Rahmen der Erfindung wird unter einem Multiolefin eine Verbindung verstanden, die mehr als eine konjugierte Kohlenstoff-Kohlenstoff-Doppelbindung aufweisen.

Beispiele von Multiolefinen umfassen Isopren, Butadien, 2-Methylbutadien, 2,4-Dimethylbutadien, Piperylen, 3-Methyl-1,3-pentadien, 2,4-hexadien, 2-neopentylbutadien, 2-Methyl-1,5-hexadien, 2,5-Dimethyl-2,4-hexadien, 2-Methyl-1,4-pentadien, 4-Butyl-1,3-pentadien, 2,3-Dimethyl-1,3-pentadien, 2,3-Dibutyl-1,3-pentadien, 2-Ethyl-1,3-pentadien, 2-Ethyl-1,3-butadien, 2-Methyl-1,6-heptadien, Cyclopentadien, Methylcyclopentadien, Cyclohexadien und 1-Vinyl-cyclohexadien.

Besonders bevorzugt ist Isopren.

Das Reaktionsmedium kann weiterhin Olefine als Monomere enthalten die weder Isoolefine noch Multiolefine sind oder nicht.

Beispiele solcher Olefine umfassen β-Pinen, Styrol, Divinylbenzol, Diisopropenylbenzol, o-, m- and p-Alkylstyrol wie o-, m- and p-Methylstyrol.

In einer Ausführungsform der Erfindung enthält das Reaktionsmedium in Schritt a) 80 Gew.-% bis 99,5 Gew.-%, vorzugsweise 85 Gew.-% bis 98,0 Gew.-%, besonders bevorzugt 85 Gew.-% bis 96,5 Gew.-% und ganz besonders bevorzugt 85 Gew.-% bis 95,0 Gew.-% an Isoolefin und von 0,5 Gew.-% bis 20 Gew.-%, bevorzugt von 2,0 Gew.-% bis 15 Gew.-%, besonders bevorzugt von 3,5 Gew.-% bis 15 Gew.-%, and ganz besonders bevorzugt von 5,0 Gew.-% bis 15 Gew.-% an Multiolefin bezogen auf das Gesamtgewicht an Monomeren im Reaktionsmedium.

In einer anderen Ausführungsform enthält das Reaktionsmedium nur Isoolefine und keine Multiolefine als Monomere.

Der Multiolefin-Gehalt von Copolymeren kann beispielsweise 0.1 mol-% oder mehr, vorzugsweise von 0.1 mol-% bis 15 mol-%, in einer anderen Ausführungsform 0.5 mol-% oder mehr, vorzugsweise 0.5 mol-% bis 10 mol-%, in einer anderen Ausführungsform 0.7 mol-% oder mehr, vorzugsweise 0.7 to 8.5 mol-%, beispielsweise 0.8 bis 3.5 mol-% oder von 4.0 bis 7.5 mol%, insbesondere für Copolymere von Isobuten und Isopren.

Der Gehalt an Monomeren im Reaktionsmedium kann beispielsweise 0.01 bis 80 Gew.-%, vorzugsweise 0.1 bis 65 Gew.-%, besonders bevorzugt von 10.0 bis 65.0 Gew.-% und ganz besonders bevorzugt 25.0 bis 65.0 Gew.-% betragen.

In einer Ausführungsform warden die Monomere vor ihrem Einsatz in Schritt A) gereinigt, insbesondere wenn sie aus einem Schritt d) recyckliert werden. Die Reinigung der Monomere kann dabei beispiesweise durch Überleiten über Absorberkolonnen mit Molsieben oder Alumina enthalteneden Absorbermaterialien erfolgen.

Um mögliche nebenreaktionen oder schädliche einflüsse auf die Reaktionsführung zu vermeiden, kann der Gehalt an Wasser, Alkoholen und anderen organischen Verbindungen mit OH-Gruppen vorzugsweise auf einen Wert von 10 Gew.-ppm oder weniger verringert werden.

### Organische Verdünnungsmittel

Der Begriff organische Verdünnungsmittel umfasst organische Verbindungen, die unter den gewählten Reaktionsbedingungen flüssig sind und für Bestandteile des Reaktionsmedium und/oder des Produktmediums verdünnende oder lösende Eigenschaften besitzen ohne mit dem Initiator-System, den Monomeren in nennenswertem Ausmass zu reagieren.

Der Begriff organische Verdünnungsmittel umfasst desweiteren auch mischungen solcher organischen Verbindungen.

Beispiele von organischen Verdünnungsmitteln umfassen Chlorkohlenwasserstoffe wie beispielsweise Methylchlroid, Methylenchlroid oder Ethylchlorid.

Weitere Beispiele von organischen Verdünnungsmitteln umfassen Fluorkohlenwasserstoffe der Formel CₓH_{y}F_{z} in der x eine ganze Zahl von 1 bis 40, vorzugsweise 3 bis 10, besonders bevorzugt 3 bis 6 ist und in der y and z ganze Zahlen sind, so dass eine der Bedingungen y+z = 2x oder y+z = 2x+2 erfüllt ist.

Weitere, bevorzugte Beispiele von organischen Verdünnungsmitteln umfassen Kohlenwasserstoffe, insbesondere aliphatische Kohlenwasserstoffe wie Propan, Isobutan, n-Pentan, Methycyclopentan, Isohexan, 2-Methylpentan, 3-Methylpentan, 2-Methylbutan, 2,2-Dimethylbutan, 2,3-Dimethylbutan, 2-Methylhexan, 3-Methylhexan, 3-Ethylpentan, 2,2-Dimethylpentan, 2,3-Dimethylpentan, 2,4-Dimethylpentan, 3,3-Dimethylpentan, 2-Methylheptan, 3-Ethylhexan, 2,5-Dimethylhexan, 2,2,4,-Trimethylpentan, n-Octan, n-Heptan, n-Butan, n-Nonan, n-Decan, n-Dodecan, n-Undecan, n-Hexan, Methyl cyclohexan, Cyclopentan, Methylcyclopentan, 1,1-Dimethylcycopentan, cis-1,2-Dimethylcyclopentan, trans-1,2-Dimethylcyclopentan, Trans-1,3-dimethyl-cyclopentan, Ethylcyclopentan, Cyclohexan und Methylcyclohexan.

Weitere Beispiele von Kohlenwasserstoffen umfassen aromatische Kohlenwasserstoffe wie Benzol, Toluol, ortho-Xylol, para- Xylol und meta-Xylol.

Besonders bevorzugte organische Verdünnungsmittel für den Einsatz in Schritt a) sind solche zumindest 90 Gew.-%, vorzugsweise zumindest 95 Gew.-% und besonders bevorzugt zumindest 98 Gew.-% an aliphatischen Kohlenwasserstoffen enthalten, die einen Siedepunkt bei 1013 hPa von -5°C bis 100°C vorzugsweise 35°C bis 85°C besitzen.

### Reaktionsbedingungen

Je nach Wahl des organischen Verdünnungsmittels und des Gewichtsanteils der Monomere wird die Polymerisation in Schritt b) in an sich bekannter Weise als sog. Slurry-Polymerisation oder Lösungspolymerisation geführt, wobei Lösungspolymerisation bevorzugt ist.

Bei einer Lösungspolymerisation bilden Monomere, das Initiator-System, und das organische Verdünnungsmittel typischerweise eine Phase, in dem auch das entstehende Polymer löslich ist.

Die Löslichkeiten der gewünschten Polymere in organischen Verdünnungsmitteln sowie ihr Quellverhalten unter den Reaktionsbedingungen sind dem Fachmann hinlänglich bekannt.

Das Initiator-System wird typischerweise in Schritt a) in einem molaren Verhältnis von Verbindungen der Formel (IIa) oder (IIb) zum Element der Gruppe IIIA, das in den Verbindungen der Formel (I) vorhanden ist, von 0,01 bis 4 eingesetzt, vorzugsweise in einem molaren Verhältnis von 0,01 bis 2.

Das Initiator-System wird typischerweise in Schritt a) in solch einer Menge eingesetzt, dass das molare Verhältnis von Element der Gruppe IIIA, das in den Verbindungen der Formel (I) vorhanden ist zu der Gesamtmenge an Monomeren von 1 bis 0,00001 vorzugsweise 0,05 bis 0,0005 beträgt.

Schritt b) kann batchweise oder kontinuierlich geführt warden, wobei eine kontinuierliche Verfahrensweise industriell bevorzugt ist.

In einer Ausführungsform kann die Polymerisation in einem polymerisationsreaktor durchgeführt warden. Dabei können alle eingesetzt werden, die dem Fachmann für diesen Zweck bekannt sind, wie zum Beispiel Durchflussreaktoren und Rohrreaktoren.

Spezifische Beispiele sind aus WO 2011/000922 A und WO 2012/089823 A bekannt.

Schritt b) wird beispielsweise bei einer Temperatur von -110 °C bis 20 °C durchgeführt, vorzugsweise -100 °C bis -20°C und besonders bevorzugt -60 °C bis -30 °C.

Der Druck in Schritt b) beträgt typischerweie 100 bis 100,000 hPa, vorzugsweise 200 bis 20,000 hPa und besonders bevorzugt 500 bis 5,000 hPa.

Diem Reaktionszeit bzw. in kontinuierlichen Verfahren die mittlere Verweilzeit in Schritt b) beträgt typischerweise 2 min bis 2 h, vorzugsweise 10 min bis 1 h und besonders bevorzugt 20 bis 45 min.

Das gewichtsmittlere Molekulargewicht M_{w} der erfindungsgemäß hergestellten Polymere beträgt beispielsweise 100 bis 2000 kg/mol, voruzugsweise 300 bis 1200 kg/mol und besonders bevorzugt 375 to 550 kg/mol. Gewichtsmittlere Molekulargewichte M_{w} werden durch GPC anhand von Polystyrolstandards ermittelt.

Vorzugsweise weisen die mit dem erfindungsgemäßen Verfahren hergestellten Polymere eine Polydispersität (PDI = Mw/Mn) von 1,0 bis 7,5, vorzugsweise von 1,0 bis 5,5, weiter bevorzugt von 1,8 bis 3,0, besonders bevorzugt 2,0 bis 3,0 auf.

Die Mooney Viskosität der erfindungsgemäß hergestellten Polymere beträgt typischerweise 10 oder mehr (ML 1 + 8 at 125°C, ASTM D 1646), vorzugsweise 10 bis 80 und besonders bevorzugt 25 bis 60.

Der Vorteil der Erfindung liegt insbesondere darin, dass Polymere bei deutlich höheren Temperaturen erzeugt werden, als dies mit üblichen aus dem Stand der Technik bekannten Initiator-Systemen der Fall ist. Insbesondere lassen sich die Polymere mit vergleichbarem Molekulargewichtsmittel bei höheren Temperaturen erzeugen, was eine signifikante Energieersparnis bewirkt. Die Stabilität der verbinduhgen der Formel (IIa) und (IIb) erlauben überdies einen wiederholten Einsatz im erfindungsgemäßen Polymerisationsverfahren.

Die vorliegende Erfindung wird durch die nachfolgenden Beispiele näher veranschaulicht.

### Beispiele

### Beispiel 1: Herstellung von 2,2"-Bis(2,3,5,6-tetrafluor-4-methoxy)-para-terphenyl

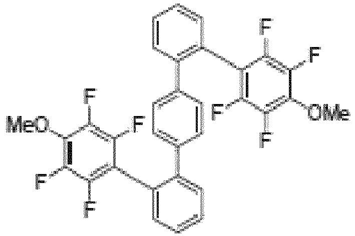

2'-Brom-2,3,5,6-tetrafluor-4-methoxybiphenyl (1,60 g, 4,78 mmol), 1,4-Phenyldiboronsäure (0,40 g, 2,33 mmol), Dioxan (12 mL) und Natriumcarbonatlösung (2 M, 6 mL) wurden 60 Minuten mit Argon entgast und anschließend Tetrakis(triphenylphosphan)palladium(0) (0,19 g, 0,16 mmol) zugegeben. Das Reaktionsgemisch wurde 24 Stunden unter Rückfluss gerührt, nach Abkühlen mit Salzsäure (10 Gew.-%ig) angesäuert und mit Dichlormethan und destilliertem Wasser aufgenommen. Die organische Phase wurde mit Salzsäure (10 Gew.-%ig), destilliertem Wasser und gesättigter Natriumchloridlösung gewaschen, über Natriumsulfat getrocknet und das Lösungsmittel unter vermindertem Druck entfernt. Das Rohprodukt wurde säulenchromatographisch (Cyclohexan / Dichlormethan (4:1), Rf = 0.31) aufgereinigt. Es wurden 0,85 g eines farblosen Feststoffs erhalten. (1,45 mmol, 62% Ausbeute, Schmelzpunkt.: 213°C)

MS (EI, 70 eV, m/z (%)): 586,2 (100) [M]^{·+}, 566,1 (20) [M - HF]⁺, 523,1 (8) [M - HF, - CO, - CH3]⁺; HR-MS (EI, m/z): 586,1177 [M]^{·+}; berechnet: 586,1174.

### Beispiel 2: Herstellung von 2,2"-Bis(2,3,5,6-tetrafluor-4-hydroxy)-para-terphenyl

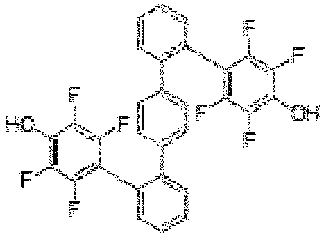

Die Verbindung aus Beispiel 1 (499 mg, 0,85 mmol) wurde in Dichlormethan (7,5 mL) gelöst und langsam Bortribromid in Dichlormethan (1 M, 4,25 mL, 4,25 mmol) zugetropft. Das Reaktionsgemisch wurde 72 h gerührt, anschließend in tert-Butylmethylether und destilliertem Wasser aufgenommen und mit Salzsäure (10 Gew.-%ig) angesäuert. Die organische Phase wird mit Salzsäure (10 Gew.-%ig), destilliertem Wasser und gesättigter Natriumchloridlösung gewaschen, die Lösung über Natriumsulfat getrocknet und das Lösungsmittel unter vermindertem Druck entfernt. Das Rohprodukt wurde über eine Filtrationssäule (Cyclohexan / THF (2:1), Rf = 0,49) vorgereinigt und anschließend mittels GPC aufgereinigt. Es wurden 311 mg eines farblosen Feststoffs erhalten.

(0,52 mmol, 61% Ausbeute, Schmelzpunkt.: 239°C)

MS (EI, 70 eV, m/z (%)): 558,2 (100) [M]^{·+} 538,1 (25) [M - HF]⁺, 518,1 (20) [M - 2HF]⁺; HR-MS (EI, m/z): 558,0864 [M]^{·+}; berechnet: 558,0861.

### Polymerisation:

### Methoden:

### Molmassenbestimmung:

Die Bestimmung der gewichtsgemittelten Molmasse erfolgt mittels Gelpermeationschromatographie (GPC). Zur Kalibrierung wurden Polystyrol-Standrads eingesetzt.

### Bestimmung des Isoprengehalts:

Zur Bestimmung des Isoprengehalts wurde durch NMR bestimmt.

### Verwendetes Reaktionsgefäß:

Das Reaktionsgefäß bestand aus einem zylindrischen Grundkörper (d=4 cm, h ≈ 6 cm) an dessen Oberseite ein senkrechter und ein seitlich, in einem Winkel von 45° angebrachter Hals mit Hülse angearbeitet sind. Acht gleichmäßig verteilte Verjüngungen (h=b=t=5 mm) waren als Strömungsstörer in einer Höhe von 10 mm des Grundkörpers eingearbeitet.

Auf die senkrechte Hülse des Reaktionsgefäßes wurde der Tropftrichter mit Druckausgleich gesetzt. Dieser bestand aus einem zylindrischen Vorratsgefäß (d=1,5 cm, h=12 cm), welches zur Kühlung mit einem weiteren zylindrischen, nach oben geöffnetem Körper (d=4,5 cm, h=11 cm) ummantelt ist. Dieser wurde mittels einer zerstoßenen Trockeneis/Acetonmischung auf -78°C gekühlt werden. Um auch bei tiefen Temperaturen die Dichtigkeit des Hahns zu gewährleisten wurde ein Eckventil mit Teflon-Spindel (Polytetrafluorethylen) verwendet.

Ein 4-Kanal-Thermometer (PCE-T390) diente zur Kontrolle der Temperatur. Dazu wurde der Temperaturfühler (Typ K) über die seitlich angebrachte Hülse durch ein Septum aus Naturkautschuk in das Reaktionsgefäß eingeführt. Zur Temperaturregulierung der Polymerisation wurde das Reaktionsgefäß in einem mit Aceton gefüllten Dewar-Gefäß mittels Kryostat der Firma THERMO SCIENTIFIC (Eintauchkühler Haake EK90) gekühlt.

Schlenk-Bedingungen wurden durch Verwendung eines Vakuumvorstoßes, welcher auf den Tropftrichter aufgesetzt wurde, erreicht. Die Schliffe der verwendeten Glasgeräte wurden mittels Teflonhülsen abgedichtet bzw. per Septum verschlossen.

**Verwendete Substanzen:**

| | | |
|---|---|---|
| n-Hexan | Lösungsmittel | ACROS, 97%, Extra Dry über Molekularsieb, AcroSeal®, ≤ 0.005% H₂O |
| Isobuten (IB) | Monomer (M) | ALDRICH, 99%), als Gas über Baylith® (LANXESS) getrocknet |
| Isopren (IP) | Monomer (M) | ALDRICH, 99%, Sure/Seal™, < 1000 ppm p-tert-Butylcatechol als Inhibitor) unter Schlenk-Bedingungen destilliert und maximal 14 Tage unter Schutzgas bei -20°C gelagert |
| Diethylaluminiumchlorid (DEAC) | Initiator (I) | ALDRICH, 1,0 M in Hexan, Sure/Seal™ |
| Ethylaluminiumdichlorid (EADC) | Initiator (I) | ALDRICH, 1,0 M in Hexan, Sure/Seal™ |
| Trimethylaluminium (TMA) | Initiator (I) | ALDRICH, 2,0 M in Hexan, Sure/Seal™ |
| Pentafluorphenol (PFP) | Coinitiator | ABCR, 99% |
| 2,2"-Bis(2,3,5,6-tetrafluor-4-hydroxy)-para-terphenyl | Coinitiator | Gemäß Beispiel 2 |

Für die Polymerisationen wurden getrocknete Glasgeräte und Magnetrührstäbchen in eine Glove-Box überführt und zusammengebaut.

### Herstellung der Monomerlösung

Zur Herstellung der 55 Vol.-%igen Monomerlösung wurden in einem graduierten Schlenkrohr n-Hexan vorgelegt und die benötigte Menge Isobuten, bei der Reaktionstemperatur einkondensiert. Das Isopren wurde in die Monomerlösung gegeben, in das Reaktionsgefäß überführt und weiterhin auf der Reaktionstemperatur gehalten.
- Monomer-Lösung:: V(Isobuten) = 12,0 ml
V(Isopren) = 0,24 ml
V(n-Hexan) = 10,0 ml

### Herstellung des Initiator-Systems

Für die Initiatorlösung wurde in einem separaten Schlenk-Rohr in der Glove-Box eine Verbindung der Formeln (IIA) oder (IIb) in n-Hexan gelöst (nachfolgend Coinitiator genannt). Die Zugabe der Verbindung der Formel (I) (nachfolgend Initiator genannt) erfolgte unter Schlenkbedingungen. Die Verweilzeit des Initiator-Systems auf Raumtemperatur betrug maximal 3 Minuten. Anschließend wurde die Lösung des Initiator-Systems 15 Minuten auf -78°C gekühlt. Unter Überwachung der Temperatur und stetigem Rühren (250 bis 375 Umdrehungen pro Minute) wurde die Lösung des Initiator-Systems innerhalb von 5 bis 10 Minuten zur Monomerlösung zugetropft. Die ursprünglich eingestellte Temperatur stieg während dem Zutropfen der Lösung des Initiator-Systems zur Monomerlösung um maximal um 10°C an.
- Initiator-System-Lösung:: n(Coinitiator) = 0,10 mmol
n(Initiator) = 0,05 mmol
V(n-Hexan) = 15,0 ml

Das molare Verhältnis von Verbindung der Formeln (IIA) oder (IIb) zu Verbindung der Formel (I) betrug demnach in allen Experimenten 2:1.

Die Polymerisation wurde für 30 Minuten durchgeführt. Anschließend wurde das Polymer in Methanol koaguliert und im Vakuum bei 50°C bis zur Gewichtskonstanz getrocknet. Die Molmassenverteilung wurde durch GPC Untersuchungen und der Isoprengehalt mittels NMR-Spektroskopie ermittelt.

Die Standardparameter sind in nachstehender Tabelle dargestellt:

**Tabelle 1**

| | |
|---|---|
| **Initiator** | DEAC |
| **Coinitiator** | PFP |
| **Monomer-Lösung** | 55 Vol.-% |
| **IP [Vol%]** | 2 |
| **T [°C]** | -70 |
| **t [min]** | 30 |
| **Coinitiator / l** | 2 |
| **c(I-Lsg) [mmol/L]** | 3,33 |
| **c(I_Vges) [mmol/L]** | 1,34 |
| **V(IB) [mL]** | 12 |
| **V(IP) [mL]** | 0,24 |
| **V(Hex_M) [mL]** | 10 |
| **n(l) [mmol]** | 0,0500 |
| **n(Coi) [mmol]** | 0,100 |
| **V(Hex_I) [ml]** | 15 |

Abweichungen von den oben angegebenen Standardparametern werden in den folgenden Beispielen explizit angeführt.

### Beispiele 4 bis 6 - Variation der Polymerisationstemperatur:

**Tabelle 1:**

| **Beispiel** | 3 | 4 | 5 |
|---|---|---|---|
| **T [°C]** | **-70** | **-50** | **-30** |
| **Polymer [g]** | 0,247 | 0,381 | 1,663 |
| **Umsatz [%]** | 3,41 | 5,26 | 22,96 |
| **M_{N}** | 493.510 | 188.100 | 122.650 |
| **M_{W}** | **913.080** | **363.040** | **238.540** |

Die Ergebnisse der Beispiele, zeigen dass selbst bei hohen Temperaturen von -50°C gleiche gewichtsgemittelte Molekulargewichte Mw für Butylkautschuk erzielt werden können, wie sie für das Initiator-System Aluminiumtrichlorid/Wasser oder HCl in Methylchlorid typischerweise nur bei -95°C erwartet werden.

### Beispiel 7 - Variation des Isoprengehalts:

**Tabelle 2:**

| **Beispiel** | 7 | 3 |
|---|---|---|
| **IP [Vol-%]** | **10** | **2** |
| **Polymer [g]** | 0,305 | 0,247 |
| **Umsatz %** | 3,86% | 3,41% |
| **M_{N}** | 93.194 | 493.510 |
| **M_{W}** | **162.110** | **913.080** |

Bei höherem Isoprengehalt verringert sich das gewichtsgemittelte Molekulargewicht Mw.

### Beispiele 8 und 9 - Variation des Initiatortyps

**Tabelle 3:**

| **#** | 8 | 9 |
|---|---|---|
| **Initiator** | **TMA** | **EADC** |
| **c(I-Lsg) [mmol/L]** | 3,33 | 0,83 |
| **c(I_Vges) [mmol/L]** | 1,34 | 0,34 |
| **Polymer [g]** | 0,003 | 0,249 |
| **Umsatz [%]** | 0,04 | 3,43 |
| **M_{N}** | 254980 | 302010 |
| **M_{W}** | **673.970** | **669.360** |
| **IP Gehalt [%]** | | |
| **n(l) [mmol]** | 0,0500 | 0,0125 |
| **n(Coi) [mmol]** | 0,100 | 0,025 |
| **V(Hex_I) [ml]** | 15 | 15 |

### Vergleichsbeispiele 10,11,12 - Änderung der Coinitiatoren

**Tabelle 4:**

| **Beispiele** | 10 | 11 | 12 |
|---|---|---|---|
| **Coinitiator** | **H₂O** | **PF-Anisol** | **Pyrazin-2,3-diol** |
| **T [°C]** | -70 | -70 | -70 |
| **Coi / l** | 2 | 2 | 1 |
| **c(I-Lsg) [mmol/L]** | 30,00 | 10,00 | 10,00 |
| **c(I_Vges) [mmol/L]** | 14,20 | 4,73 | 4,73 |
| **Polymer [g]** | - | - | - |
| **Umsatz %** | | | |
| **M_{N}** | - | --- | - |
| **M_{W}** | - | - | - |
| **Kommentar** | InitiatorLösung: weißer Niederschlag (Aluminiumoxid ?) | Initiator-Lösung: keine Verfärbu ng | Pyrazindi ol in DCM nicht komplett löslich |
| **V(IB) [mL]** | 3 | 3 | 3 |
| **V(IP) [mL]** | 0,06 | 0,06 | 0,06 |
| **V(Hex_M) [mL]** | 2,5 | 2,5 | 2,5 |
| **n(I) [mmol]** | 0,1500 | 0,0500 | 0,0500 |
| **n(Coi) [mmol]** | 0,300 | 0,100 | 0,050 |
| **V(Hex_I) [ml]** | 5 | 5 | 0 |
| **V(DCM_I) [ml]** | 0 | 0 | 5 |

## Patentansprüche

1. Initiator- System umfassend
I) zumindest eine Verbindung der Formel (I)
AY¹Y²Y³ (I)
in der
A für ein Metall aus der Gruppe IIIA, vorzugsweise Aluminium, Bor, Gallium oder Indium, besonders bevorzugt Bor und Aluminium und besonders bevorzugt Aluminium steht und
Y¹, Y² und Y³ jeweils unabhängig voneinander Fluor, Chlor, Brom, lod oder (C₁-C₁₈)-Alkyl stehen können und
II) zumindest eine Verbindung der Formeln (IIa) oder (IIb) 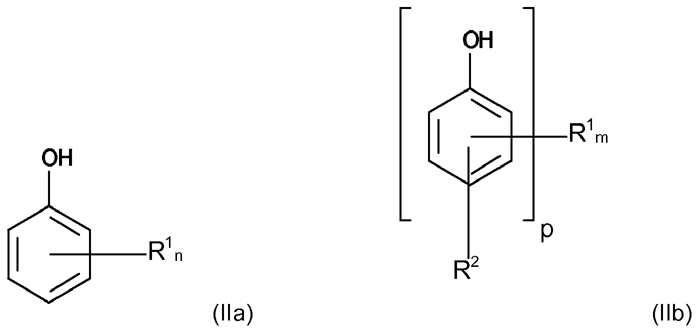 in denen
n 1, 2, 3, 4 oder 5 und
m 1, 2, 3 oder 4 und
p 2, 3 oder 4 sein können
R¹ unabhängig von gegebenenfalls weiteren Resten R¹ ausgewählt ist aus der Gruppe Fluor, Chlor, Hydroxy, CN, NO₂, (C₁-C₁₈)-Alkyl, (C₁-C₁₈)-Alkoxy, (C₆-C₂₄)-Alkylaryl, (C₆-C₂₄)-Arylalkyl oder (C₆-C₁₀)-Aryl und
R² für eine Bindung (in diesem Fall ist p =2) oder einen p-valenten Rest steht, der sich von Benzol, Naphthalin, Biphenyl, Terphenyl oder Tetraphenyl ableitet und der nicht, einfach oder mehrfach unabhängig weiter substituiert sein kann mit Resten aus der Gruppe Halogen, Hydroxy, CN, NO₂, (C₁-C₁₈)-Alkyl, (C₁-C₁₈)-Alkoxy, (C₆-C₂₄)-Alkylaryl oder (C₆-C₂₄)-Arylalkyl.

2. Initiator-System nach Anspruch 1, **dadurch gekennzeichnet, dass** in Formel (I) Y¹, Y² und Y³, bevorzugt jeweils unabhängig voneinander, für Chlor oder (C₁-C₁₈)-Alkyl stehen.

3. Initiator-System nach Anspruch 1 oder 2, **dadurch gekennzeichnet, dass** in Formel (I) Y¹, Y² und Y³ jeweils unabhängig voneinander Chlor oder C₁-C₄-Alkyl, insbesondere für Chlor, Methyl oder Ethyl und ganz besonders für Chlor oder Ethyl stehen.

4. Initiator-System nach einem der Ansprüche 1 bis 3, **dadurch gekennzeichnet, dass** als Verbindungen der Formel (I) Ethylaluminiumdichlorid (EADC) und Diethylaluminiumchlorid (DEAC) oder Mischungen davon eingesetzt werden.

5. Initiator-System nach einem der Ansprüche 1 bis 4, **dadurch gekennzeichnet, dass** in den Formeln (IIa) und (IIb) R¹ für Fluor, Chlor, Hydroxy, (C₁-C₄)-Alkyl oder (C₁-C₄)-Alkoxy, besonders bevorzugt Fluor oder (C₁-C₄)-Alkyl, ganz besonders bevorzugt für Fluor stehen.

6. Initiator-System nach einem der Ansprüche 1 bis 5, **dadurch gekennzeichnet, dass** als Komponente II) Pentafluorphenol eingesetzt wird.

7. Verfahren zur Herstellung von Verbindungen der Formel (IIb) durch
A) Umsetzung von Verbindungen der Formel (III) 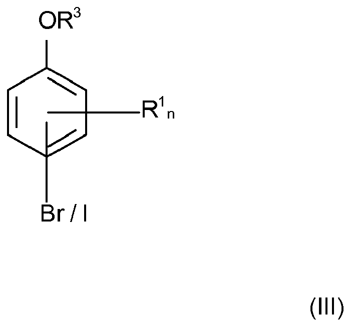 mit Verbindungen der Formel (IV) oder (V) 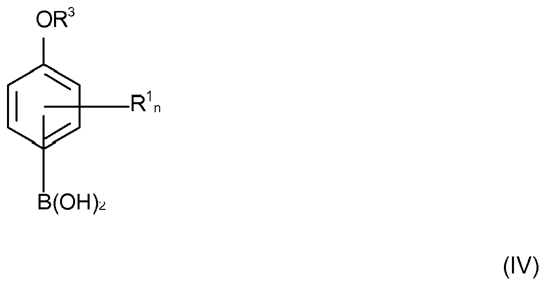
R²[B(OH)₂]ₚ (V)
in Gegenwart von Palladium enthaltenden Katalysatoren wie beispielsweise Tetrakis(triphenylphosphan)palladium(0)
und anschließend
B) Spaltung der Ether-gruppen z.B. mit Bortribromid, Bortrichlorid oder Chloroder Bromwasserstoff.

8. Verbindungen der Formel (IIb) gemäß Anspruch 1.

9. 2,2"-Bis(2,3,5,6-tetrafluor-4-hydroxy)-para-terphenyl.

10. Verfahren zur Herstellung von Homo- oder Copolymeren, die Wiederholungseinheiten aufweisen sich sich von Isoolefinen ableiten, **dadurch gekennzeichnet**, das zur Initiierung der Polymerisation ein Initiatorsystem gemäß einem der Ansprüche 1 bis 6 eingesetzt wird.

11. Verfahren nach Anspruch 10, **dadurch gekennzeichnet, dass** es zumindest die folgenden Schritte umfasst:
a) Bereitstellung eines Reaktionsmediums enthaltend ein organisches Verdünnungsmittel und zumindest Isoolefin als Monomer, vorzugsweise zumindest ein Isoolefin und zumindest ein Multiolefin als Monomere;
b) Polymerisation der Monomere im Reaktionsmedium in Gegenwart des Initiator-Systems zur Erzeugung eines Produktmediums umfassend das Polymer, das organische Verdünnungsmittel und gegebenenfalls unreagierte Monomere und optional
c) Abtrennung des Polymers aus dem Produktmedium.

12. Verfahren nach Anspruch 11, **dadurch gekennzeichnet, dass** als Isoolefine solche aus der Gruppe bestehend aus Isobuten, 2-Methyl-1-buten, 3-Methyl-1-buten und 2-Methyl-2-buten ausgewählt werden, wobei Isobuten bevorzugt ist.

13. Verfahren nach einem der Ansprüche 11 oder 12, **dadurch gekennzeichnet, dass** als Multiolefine solche aus der Gruppe bestehend aus Isopren, Butadien, 2-Methylbutadien, 2,4-Dimethylbutadien, Piperylen, 3-Methyl-1,3-pentadien, 2,4-hexadien, 2-neopentylbutadien, 2-Methyl-1,5-hexadien, 2,5-Dimethyl-2,4-hexadien, 2-Methyl-1,4-pentadien, 4-Butyl-1,3-pentadien, 2,3-Dimethyl-1,3-pentadien, 2,3-Dibutyl-1,3-pentadien, 2-Ethyl-1,3-pentadien, 2-Ethyl-1,3-butadien, 2-Methyl-1,6-heptadien, Cyclopentadien, Methylcyclopentadien, Cyclohexadien und 1-Vinyl-cyclohexadien ausgewählt werden, wobei Isopren bevorzugt ist.

14. Verfahren nach einem der Ansprüche 11 bis 13, **dadurch gekennzeichnet, dass** als organisches Verdünnungsmittel Chlorkohlenwasserstoffe wie beispielsweise Methylchlorid, Methylenchlorid oder Ethylchlorid, Fluorkohlenwasserstoffe der Formel CₓH_{y}F_{z} in der x eine ganze Zahl von 1 bis 40, vorzugsweise 3 bis 10, besonders bevorzugt 3 bis 6 ist und in der y and z ganze Zahlen sind, so dass eine der Bedingungen y+z = 2x oder y+z = 2x+2 erfüllt ist oder Kohlenwasserstoffe, wie insbesondere aliphatische Kohlenwasserstoffe wie Propan, Isobutan, n-Pentan, Methycyclopentan, Isohexan, 2-Methylpentan, 3-Methylpentan, 2-Methylbutan, 2,2-Dimethylbutan, 2,3-Dimethylbutan, 2-Methylhexan, 3-Methylhexan, 3-Ethylpentan, 2,2-Dimethylpentan, 2,3-Dimethylpentan, 2,4-Dimethylpentan, 3,3-Dimethylpentan, 2-Methylheptan, 3-Ethylhexan, 2,5-Dimethylhexan, 2,2,4,-Trimethylpentan, n-Octan, n-Heptan, n-Butan, n-Nonan, n-Decan, n-Dodecan, n-Undecan, n-Hexan, Methyl cyclohexan, Cyclopentan, Methylcyclopentan, 1,1-Dimethylcycopentan, cis-1,2-Dimethylcyclopentan, trans-1,2-Dimethylcyclopentan, Trans-1,3-dimethyl-cyclopentan, Ethylcyclopentan, Cyclohexan und Methylcyclohexan oder aromatische Kohlenwasserstoffe wie Benzol, Toluol, ortho-Xylol, para- Xylol und meta-Xylol oder mischungen aus den vorgenannten organischen Verdünnungsmitteln eingesetzt werden.

15. Verfahren nach einem der Ansprüche 11 bis 14, **dadurch gekennzeichnet, dass** das Initiator-System in Schritt a) in solch einer Menge eingesetzt wird, dass das molare Verhältnis von Element der Gruppe IIIA, das in den Verbindungen der Formel (I) vorhanden ist zu der Gesamtmenge an Monomeren von 1 bis 0,00001 vorzugsweise 0,05 bis 0,0005 beträgt.
